# EUROPÄISCHE PATENTSCHRIFT

(11) **EP 1 948 585 B1**
(45) Veröffentlichungstag und Bekanntmachung des Hinweises auf die Patenterteilung: **18.03.2009**
(21) Anmeldenummer: 06807659.5
(22) Anmeldetag: 30.10.2006
(51) Int. Cl.: C07C 67/05, C07C 69/15

(54) **VERFAHREN ZUR HERSTELLUNG VON VINYLACETAT UNTER NUTZUNG DER DABEI FREIWERDENDEN REAKTIONSWÄRME**
PROCESS FOR PREPARATION OF VINYL ACETATE USING THE HEAT OF REACTION LIBERATED IN THE PROCESS
PROCEDE DE FABRICATION D ACETATE DE VINYLE UTILISANT LA CHALEUR DE REACTION LIBEREE

(30) Priorität: 15.11.2005 DE 102005054411
(43) Veröffentlichungstag der Anmeldung: 30.07.2008
(73) Patentinhaber: Wacker Chemie AG, 81737 München (DE)
(72) Erfinder: MICHL, Harald, 84453 Mühldorf (DE)
(74) Vertreter: Schuderer, Michael
(86) Internationale Anmeldenummer: PCT/EP2006/067933
(87) Internationale Veröffentlichungsnummer: WO 2007/057297

(56) Entgegenhaltungen:
- WO-A-20/05092829
- DATABASE WPI Week 199019 Derwent Publications Ltd., London, GB; AN 1990-144324 XP002420435 & JP 02 091044 A (NIPPON SYNTHETIC CHEM IND CO) 30. März 1990 (1990-03-30) in der Anmeldung erwähnt

## Beschreibung

Die Erfindung betrifft ein Verfahren zur Herstellung von Vinylacetat in einem heterogen katalysierten, kontinuierlichen Gasphasenprozess mittels Umsetzung von Ethylen mit Essigsäure und Sauerstoff, unter Nutzung der dabei freiwerdenden Reaktionswärme.

Vinylacetat wird in kontinuierlichen Verfahren unter Rückführung des aufgereinigten Produktstromes hergestellt. In einem heterogen katalysierten Gasphasenprozess reagiert Ethylen mit Essigsäure und Sauerstoff an Festbett- oder Wirbelschichtkatalysatoren, welche im allgemeinen Palladium und Alkalimetallsalze auf einem Trägermaterial enthalten und zusätzlich noch mit Gold, Rhodium oder Cadmium dotiert sein können.

Die Edukte Ethylen, Sauerstoff und Essigsäure werden in einer exothermen Reaktion im allgemeinen bei einem Druck von 1 bis 30 bar (Druckangaben hier und im folgenden in bar Überdruck) und einer Temperatur von 130°C bis 200°C in einem Festbettröhren- oder Wirbelschichtreaktor zu Vinylacetat umgesetzt: C₂H₄ + CH₃COOH + 0.5 O₂ => CH₃COOCH=CH₂ + H₂O Der Ethylenumsatz beträgt dabei etwa 10 %, der Essigsäureumsatz etwa 20 bis 30 % und der Sauerstoffumsatz bis zu 90 %.

Bei der Herstellung von Vinylacetat wird ein vorwiegend aus Ethylen, Kohlendioxid, Ethan, Stickstoff und Sauerstoff bestehendes Gasgemisch (Kreisgas mit in der Regel 60 bis 70 Vol.-% Ethylen) im Kreis geführt. Der Gasstrom wird vor dem Festbettröhren- oder Wirbelschichtreaktor mit den Reaktanden Essigsäure, Ethylen und Sauerstoff versetzt und mit Heizdampf betriebenen Wärmetauschern auf Reaktionstemperatur gebracht. Die Anreicherung des Kreisgases mit Essigsäure erfolgt üblicherweise mittels einem mit Heizdampf geheizten Essigsäuresättiger oder Essigsäureverdampfer. Nach der Reaktion werden die Reaktionsprodukte und nicht umgesetzte Essigsäure aus dem

Kreisgas auskondensiert und der Aufarbeitung zugeführt. Nicht auskondensiertes Produkt wird in einem mit Essigsäure betriebenen Wäscher ausgewaschen.

Das Kreisgas oder eine Teilmenge desselben wird, bevor es erneut mit den Edukten versetzt wird, vom gebildeten Kohlendioxid gereinigt.

Die auskondensierten Produkte Vinylacetat und Wasser sowie nicht umgesetzte Essigsäure werden in einem mehrstufigen, üblicherweise mit Heizdampf betriebenem, Destillationsprozess voneinander getrennt. Die üblichen Destillationsschritte sind Entwässerung, Azeotropdestillation, Reindestillation, Nebenproduktabtrennung, Abwasserreinigung, Rückstandsaufarbeitung sowie Leichtsieder- und Hochsieder-Abtrennung. Bezüglich der Aufarbeitung des Vinylacetats können sich die Produktionsanlagen unterscheiden.

Die Reaktionstemperatur im Festbettröhren- oder Wirbelschichtreaktor von 130°C bis 200°C wird mittels Siedewasserkühlung bei einem Druck von 1 bis 10 bar eingestellt. Dabei wird Wasserdampf, der sogenannte Eigendampf, mit einer Temperatur von 120°C bis 185°C bei einem Druck von 1 bis 10 bar, vorzugsweise 2,5 bis 5 bar, gebildet. Unter Umständen kann der Dampf etwas überhitzt sein. In diesem Fall ist die Temperatur höher als die Siedetemperatur bei dem jeweiligen Eigendampfdruck. Dieser Eigendampf kann dann zur Beheizung von weiteren Prozeßschritten der Vinylacetat-Herstellung eingesetzt werden, beispielsweise zur Beheizung von einzelnen Destillationskolonnen zur Auftrennung des Produktgemisches. Eine solche Vorgehensweise ist in der JP-A 02-091044 beschrieben.

Die Reaktionstemperatur wird über den Betriebsdruck der Siedewasserkühlung und des entstehenden Eigendampf eingestellt. Die über die Betriebszeit eines Katalysators nachlassende Aktivität wird durch Erhöhen der Reaktionstemperatur, das heißt des Betriebsdrucks der Siedewasserkühlung und des entstehenden Eigendampfs ausgeglichen. Die Reaktions- und somit die Eigendampf-Temperatur ist somit zeitlich unterschiedlich, was zu Verbringungsproblemen des Eigendampfs führt. Es gilt hierbei, dass zum Schonen des Katalysators, zum Optimieren der Selektivität und zur Minimierung der Kohlendioxid-Bildung die Vinylacetat-Reaktion möglichst lange mit einer niedrigen Reaktionstemperatur, sprich niedrigem Eigendampfdruck betrieben wird.

Nachteilig ist dabei, dass der Eigendampf, aufgrund dessen niedrigem Temperatur- und Druckniveaus, nur zur Beheizung eines Teils der Prozeßschritte eingesetzt werden kann. Dies sind beispielsweise die Entwässerungskolonne, Abwasserreinigung, die üblicherweise unter Vakuum betriebene Rückstandsaufarbeitung, ein Kreisgas-Erwärmer sowie verschiedene EssigsäureVerdampfer und -Erhitzer. Für die weiteren Prozeßschritte wie Azeotropdestillation oder Reindestillation muß externer, höherwertiger und oft überhitzter Heizdampf zugeführt werden, üblicherweise mit einer Temperatur von 160°C bis 250°C und einem Druck von 5 bar bis 15 bar. Ein weiterer Nachteil besteht darin, dass im Prozeß bei der Reaktorkühlung der exothermen Gasphasenreaktion mehr Eigendampf anfällt, als in den Prozeßschritten der Vinylacetat-Herstellung und -Reinigung aufgrund des Druck- und Temperaturniveaus des Eigendampfs verbraucht werden kann. Es können üblicherweise nur etwa 75 bis 80 Gew.-% des gebildeten Eigendampfs zur Beheizung in Prozeßschritten verbraucht werden. Die Verwertung des Eigendampfs für Prozeßschritte hängt stark von den gewählten Apparatedimensionen und des Druckniveaus des verwendeten Heizdampfs für den Anlagenbetrieb ab.

Der Restanteil kann entweder kondensiert werden, was zu vollständigem Energieverlust führt, oder alternativ dazu im Rahmen eines Werkverbundes an andere Betriebe abgegeben werden. Dies ist jedoch mit einem organisatorischen und apparativen Aufwand verbunden. Zudem wird Niederdruckdampf meist für die Beheizung von ausgewählten Produktrohrleitungen oder Gebäuden eingesetzt, unterliegt damit jahreszeitlichen Schwankungen und kann folglich oftmals nicht vollständig weiterverwendet werden.

Es bestand daher die Aufgabe, ein Verfahren zur Herstellung von Vinylacetat unter Nutzung der dabei freiwerdenden Reaktionswärme zur Verfügung zu stellen, bei dem der bei der exothermen Gasphasenreaktion gebildete Eigendampf vollständig bei der Vinylacetat-Herstellung weiterverwendet werden kann.

Gegenstand der Erfindung ist ein Verfahren zur Herstellung von Vinylacetat mittels
a) heterogen katalysierter, kontinuierlicher Gasphasenreaktion von Ethylen, Essigsäure und Sauerstoff, bei einem Druck von 1 bis 30 bar und einer Temperatur von 130°C bis 200°C, wobei die Prozesswärme mittels Wärmetausch an Wasser mit einer Temperatur von 120°C bis 185°C bei einem Druck von 1 bis 10 bar abgeführt wird,
b) Auftrennung des Produktgasstromes enthaltend im wesentlichen Ethylen, Vinylacetat, Essigsäure, Wasser, Kohlendioxid und weitere Inertgase, und
c) ganz oder teilweiser Rückführung von Ethylen in den Kreisgasprozess,
dadurch gekennzeichnet, dass
der bei der Gasphasenreaktion mittels Wärmetausch gebildete Wasserdampf (Eigendampf) mit einer Temperatur von 120°C bis 185°C bei einem Druck von 1 bis 10 bar, ganz oder teilweise, um einen Differenzdruck von mindestens 0,5 bar verdichtet und weiterverwendet wird.

Bei der kontinuierlichen Herstellung von Vinylacetat wird in Röhrenreaktoren gearbeitet, welche mit einem Festbettkatalysator beschickt sind, oder in Wirbelschichtreaktoren mit für die Wirbelschicht geeigneten Katalysatoren. Als Katalysatoren finden im allgemeinen mit Edelmetall(salz)en und Promotoren dotierte Trägerkatalysatoren, beispielsweise mit Palladiumchlorid und mit Au-, Cd- und K-Salzen dotierte Bentonit-Kugeln Verwendung. Der Reaktor wird mit Ethylen, Sauerstoff und Essigsäure beschickt und die Reaktion vorzugsweise bei einem Druck von 8 bis 12 bar und einer Temperatur von 130°C bis 170°C durchgeführt. Druck und Temperatur des erzeugten Eigendampfs liegen bei den bevorzugten Reaktionsbedingungen üblicherweise bei 2,5 bis 5 bar und 140°C bis 160°C.

Der aus dem Reaktor austretende Produktgasstrom enthält im wesentlichen Vinylacetat, Ethylen, Essigsäure, Wasser, Sauerstoff, Kohlendioxid sowie die Inerten Stickstoff, Argon, Methan und Ethan.

Die Reaktionsprodukte werden mit der im Überschuß zugegebenen Essigsäure aus dem Produktgasstrom bei Systemdruck auskondensiert und in anschließenden Wäscherstufen mit Essigsäure ausgewaschen. Vinylacetat, Essigsäure, Wasser und weitere kondensierbare Anteile werden anschließend destillativ voneinander getrennt.

Der bei der Gasphasenreaktion mittels Wärmetausch gebildete Wasserdampf (Eigendampf) mit einer Temperatur von 120°C bis 185°C bei einem Druck von 1 bis 10 bar oder Teilmengen davon werden anschließend verdichtet. Die Verdichtung kann mittels mechanischer Verdichter erfolgen. Diese verdichten so, dass keine Kondensation des Dampfes durch die Druckerhöhung stattfindet, beispielsweise durch Vorschalten eines heizbaren Wärmetauschers, der den Dampf zur Verhinderung von Kondensation überhitzt.

Bevorzugt erfolgt die Verdichtung des Eigendampfes mittels gängiger Dampfstrahl-Dampfkompressoren (Injektordüsen). Dies hat den Vorteil, dass Hochdruckdampf, welcher bei der Herstellung von Vinylacetat zum Anwärmen des Reaktionsgases vor dem Reaktor ohnehin eingesetzt wird, verwendet werden kann. Die Problematik der Kondensation ist nicht vorhanden und der maschinelle und apparatelle Aufwand ist gering. Der durch den Dampfstrahl-Dampfkompressor tretende Hochdruckdampf saugt dabei Eigendampf an und verdichtet diesen. Das resultierende Druckniveau ist niedriger als das Druckniveau des Hochdruck-Treibdampfes. Die Verdichtung erfolgt nahezu isentrop (adiabat), so dass sich der Gesamtwärmeinhalt aus der Summe der Wärmeinhalte der beiden vermischten Dämpfe ergibt.

Besonders bevorzugt ist die Verwendung von geregelten Dampfstrahl-Dampfkompressoren, da diese der Eigendampfmenge und dem Druckniveau des zu verdichtenden Eigendampfes angepasst werden können. Darüberhinaus ist mehr Flexibilität hinsichtlich des zu erreichenden Enddrucks möglich. Dampfstrahl-Dampfkompressoren arbeiten im allgemeinen nach dem Venturi-Prinzip. Dabei wird der Hochdruck-Treibdampf in den Dampfstrahl-Dampfkompressor und in diesem über eine in der Strahlpumpe geregelte Treibdüse geleitet. Über eine weitere Zuleitung wird Eigendampf dem Dampfstrahl-Dampfkompressor zugeführt. Der Mischdampf tritt aus einer Mischdampfleitung aus dem Dampfstrahl-Dampfkompressor aus, wobei mittels eines Manometers dessen Druck ermittelt wird. Das Manometer und die Regelung der Treibdüse sind über einen Regelkreis verbunden, so dass der gewünschte Enddruck des Mischdampfes über das Regelventil bei der Zuleitung des Hochdruck-Treibdampfes eingestellt werden kann. Alternativ kann auch die zugeführte Treibdampfmenge die Treibdüse regeln. Die Menge an Treibdampf muß so groß sein, daß die Eigendampfmenge auf den gewünschten Druck der Mischung verdichtet wird.

Alternativ wird der Hochdruck-Treibdampf über ein Regelventil in den Dampfstrahl-Dampfkompressor geleitet. Über eine weitere Zuleitung wird Eigendampf dem Dampfstrahl-Dampfkompressor zugeführt. Der Mischdampf tritt aus einer Mischdampfleitung aus dem Dampfstrahl-Dampfkompressor aus, wobei mittels eines Manometers dessen Druck ermittelt wird. Das Manometer und das Regelventil sind über einen Regelkreis verbunden, so dass der gewünschte Enddruck des Mischdampfes über das Regelventil bei der Zuleitung des Hochdruck-Treibdampfes eingestellt werden kann. Auch hier kann die zugeführte Treibdampfmenge die Treibdüse regeln. Die Menge an Treibdampf muß so groß sein, dass die Eigendampfmenge auf den gewünschten Druck der Mischung verdichtet wird.

Der bei der Verdichtung angestrebte Enddruck des Eigendampfes liegt im allgemeinen um einen Differenzdruck von mindestens 0,5 bar über dem Druck des Eigendampfes. Vorzugsweise liegt der bei der Verdichtung angestrebte Enddruck des Eigendampfes um einem Differenzdruck von bis zu 10 bar über dem Druck des Eigendampfes, besonders bevorzugt bis zu 5 bar und am meisten bevorzugt bis zu 3 bar über dem Eintrittsdruck des Eigendampfes.

Vorzugsweise wird der Eigendampf auf ein Druckniveau von bis zu 15 bar, besonders bevorzugt auf ein Druckniveau von 5 bis 10 bar verdichtet.

Bei der Verdichtung des Eigendampfes mittels Dampfstrahl-Dampfkompressoren liegt der Druck des Hochdruck-Treibdampfes mindestens 0,5 bar über dem Druck des Eigendampfes, vorzugsweise bis zu 10 bar, besonders bevorzugt bis zu 20 bar über dem Druck des Eigendampfes. Das Mischungsverhältnis von Hochdruck-Treibdampf zu Eigendampf hängt vom Druckniveau der beiden Dampfströme und dem gewünschten Druck der Mischung ab. Das Gewichtsverhältnis ist im allgemeinen kleiner 8 : 1, bevorzugt kleiner 5 : 1 und besonders bevorzugt kleiner 3 : 1.

Aufgrund der Kostenstruktur von modernen Strom-Wärme-Kraftwerken ist der Kostenunterschied zwischen Heizdampf niederer Druckstufe, etwa von 5 bar, gegenüber Heizdampf relativ hoher Druckstufe, etwa von 15 bar, gering. Damit leitet sich der Vorteil ab, die Eigendampfverdichtung mit Treibdampf relativ hoher Druckstufe, zum Beispiel 15 bar, zu betreiben, um so möglichst wenig Treibdampf zu benötigen und somit die Kosten für die von extern zu beziehenden Heizdampfmengen zu minimieren.

Mit dem erfindungsgemäßen Verfahren wird die teilweise oder vollständige Nutzung des über die Vinylacetat-Reaktionswärme erzeugten Eigendampfes ermöglicht. Der verdichtete Eigendampf kann im Vinylacetat-Prozess wiederverwendet werden oder in anderen Verfahren verwendet werden. Der Bezug von externen Heizdampf für den Vinylacetat-Herstellungsprozeß kann somit deutlich minimiert werden.

Die nachfolgenden Beispiele dienen zur weiteren Erläuterung der Erfindung:

### Vergleichsbeispiel 1:

Ein Röhrenreaktor, welcher mit einem Pd/Au-Trägerkatalysator ausgerüstet war, wurde bei einem Druck von 9,5 bar und einer Temperatur von 160°C mit einem Ethylen, Essigsäure und Sauerstoff enthaltenden Gasgemisch beschickt.

Der Reaktor wurde mit Siedewasserkühlung betrieben. Aus der umlaufenden Wassermenge wurden 26,4 to/h Eigendampf mit einem Druck von 3,6 bar gebildet.

Aus dem aus dem Reaktor austretenden Kreisgas wurden das gebildete Vinylacetat, Wasser und die überschüssige Essigsäure abgetrennt und in der nachfolgenden Destillation voneinander getrennt, und auch Nebenprodukte, Leicht- und Hochsieder abdestilliert. Das aufgereinigte Kreisgas wurde nach Abtrennen des Nebenprodukts Kohlendioxid und erneutem Zumischen von Essigsäure, Ethylen und Sauerstoff in den Reaktor zurückgeführt.

Der bei Siedewasserkühlung entstandene 3,6 bar Eigendampf wurde zur Beheizung der Entwässerungskolonne, der Abwasserreinigung, einer Nebenproduktkolonne, der Rückstandsaufarbeitung, eines Kreisgaserwärmers, eines Verdampfers der Kohlendioxidabtrennung aus dem Kreisgas sowie weiterer Wärmetauscher der Anlage eingesetzt.

Es wurden 6,5 to/h ungenutzter Eigendampf aus der Anlage an andere Produktionsbetriebe abgegeben. Zur Beheizung der weiteren Anlagenteile der Vinylacetat-Produktionsanlage wurden zusätzlich noch 19,5 to/h 5 bar Heizdampf und 4,4 to/h 15 bar Heizdampf benötigt.

Die von extern zugeführte Heizdampfmenge betrug folglich 23,9 to/h.

### Beispiel 2:

Es wurde analog Beispiel 1 vorgegangen, mit dem Unterschied, dass die 6,5 to/h in der Anlage ungenutzen Eigendampfs mit einem Druck von 3,6 bar mittels eines Dampfstrahl-Dampfkompressors und 8,6 to/h überhitzten 15-bar-Treibdampf mit 248°C auf 5 bar und 193°C verdichtet wurden. Der verdichtete Eigendampf wurde zusätzlich zur Beheizung der Azeotrop- und Reinkolonne eingesetzt.

Der verdichtete Eigendampf wurde vollständig in der Anlage genutzt.

Es wurden zusätzlich noch 4,4 to/h 15 bar Heizdampf zum Beheizen der Anlage zugeführt. In Summe betrug der Bedarf an 15 bar Heizdampf 13 to/h. Es bestand kein Bedarf mehr an 5 bar Heizdampf zur Beheizung innerhalb der Anlage.

Die von extern zugeführte Heizdampfmenge reduzierte sich gegenüber Vergleichsbeispiel 1 um 10,9 to/h auf 13 to/h.

## Patentansprüche

1. Verfahren zur Herstellung von Vinylacetat mittels
a) heterogen katalysierter, kontinuierlicher Gasphasenreaktion von Ethylen, Essigsäure und Sauerstoff, bei einem Druck von 1 bis 30 bar und einer Temperatur von 130°C bis 200°C, wobei die Prozesswärme mittels Wärmetausch an Wasser mit einer Temperatur von 120°C bis 185°C bei einem Druck von 1 bis 10 bar abgeführt wird,
b) Auftrennung des Produktgasstromes enthaltend im wesentlichen Ethylen, Vinylacetat, Essigsäure, Wasser, Kohlendioxid und weitere Inertgase, und
c) ganz oder teilweiser Rückführung von Ethylen in den Kreisgasprozess, **dadurch gekennzeichnet, dass**
der bei der Gasphasenreaktion mittels Wärmetausch gebildete Wasserdampf mit einer Temperatur von 120°C bis 185°C bei einem Druck von 1 bis 10 bar, ganz oder teilweise, um einen Differenzdruck von mindestens 0,5 bar verdichtet wird und weiterverwendet wird.

2. Verfahren nach Anspruch 1, **dadurch gekennzeichnet, dass** der bei der Gasphasenreaktion mittels Wärmetausch gebildete Wasserdampf auf ein Druckniveau von bis zu 15 bar verdichtet wird.

3. Verfahren nach Anspruch 1 oder 2, **dadurch gekennzeichnet, dass** die Verdichtung mittels mechanischer Verdichter oder mittels Dampfstrahl-Dampfkompressoren erfolgt.

4. Verfahren nach Anspruch 3, **dadurch gekennzeichnet, dass** bei der Verdichtung mittels Dampfstrahl-Dampfkompressoren Hochdruckdampf, welcher bei der Herstellung von Vinyl-acetat zum Anwärmen des Reaktionsgases vor dem Reaktor eingesetzt wird, verwendet wird.

5. Verfahren nach Anspruch 1 bis 4, **dadurch gekennzeichnet, dass** der verdichtete Eigendampf im Vinylacetat-Prozess wiederverwendet wird.

## Claims

1. Process for preparing vinyl acetate by means of
a) a heterogeneously catalyzed, continuous gas-phase reaction of ethylene, acetic acid and oxygen at a pressure of from 1 to 30 bar and a temperature of from 130°C to 200°C, with the process heat being removed by heat exchange with water at a temperature of from 120°C to 185°C and a pressure of from 1 to 10 bar,
b) fractionation of the product gas stream consisting essentially of ethylene, vinyl acetate, acetic acid, water, carbon dioxide and further inert gases, and
c) recirculation of all or part of the ethylene to the recycle gas process, **characterized in that** all or part of the steam formed in the gas-phase reaction by heat exchange and having a temperature of from 120°C to 185°C and a pressure of from 1 to 10 bar is compressed by a differential pressure of at least 0.5 bar and used further.

2. Process according to Claim 1, **characterized in that** the steam formed in the gas-phase reaction by heat exchange is compressed to a pressure level of up to 15 bar.

3. Process according to Claim 1 or 2, **characterized in that** compression is effected by means of mechanical compressors or by means of steam-jet steam compressors.

4. Process according to Claim 3, **characterized in that** high-pressure steam which is used in the preparation of vinyl acetate for preheating the reaction gas upstream of the reactor is used in the compression by means of steam-jet steam compressors.

5. Process according to any of Claims 1 to 4, **characterized in that** the compressed process-generated steam is used further in the vinyl acetate process.

## Revendications

1. Procédé pour la préparation d'acétate de vinyle par
a) réaction en phase gazeuse catalysée par voie hétérogène, continue, d'éthylène, d'acide acétique et d'oxygène, à une pression de 1 à 30 bars et une température de 130°C à 200°C, la chaleur de procédé étant évacuée par échange thermique via de l'eau à une température de 120°C à 185°C à une pression de 1 à 10 bars,
b) séparation du flux de gaz produit contenant essentiellement de l'éthylène, de l'acétate de vinyle, de l'acide acétique, de l'eau, du dioxyde de carbone et d'autres gaz inertes, et
c) recyclage total ou partiel de l'éthylène dans le processus gazeux en circulation,
**caractérisé en ce que** la vapeur d'eau formée lors de la réaction en phase gazeuse par échange thermique présentant une température de 120°C à 185°C à une pression de 1 à 10 bars, est comprimée totalement ou partiellement, à une pression différentielle d'au moins 0,5 bar et est utilisée ultérieurement.

2. Procédé selon la revendication 1, **caractérisé en ce que** la vapeur d'eau formée lors de la réaction en phase gazeuse par échange thermique est comprimée à un niveau de pression allant jusqu'à 15 bars.

3. Procédé selon la revendication 1 ou 2, **caractérisé en ce que** la compression est réalisée au moyen de compresseurs mécaniques ou de compresseurs de vapeur à jet de vapeur.

4. Procédé selon la revendication 3, **caractérisé en ce qu'**on utilise lors de la compression au moyen de compresseurs de vapeur à jet de vapeur, de la vapeur à haute pression, qui est utilisée lors de la préparation d'acétate de vinyle pour le chauffage du gaz de réaction en amont du réacteur.

5. Procédé selon la revendication 1 à 4, **caractérisé en ce que** la propre vapeur compressée est réutilisée dans le processus d'acétate de vinyle.
